Europäisches Patentamt

**European Patent Office**

Office européen des brevets

(11) Numéro de publication: **0 086 686**
**B1**

(12) FASCICULE DE BREVET EUROPEEN

(45) Date de publication du fascicule du brevet: **02.10.85**

(51) Int. Cl.⁴: **A 61 L 15/07, C 08 L 67/04**

(21) Numéro de dépôt: **83400144.8**

(22) Date de dépôt: **20.01.83**

(54) **Nouvelle composition thermoplastique à mémoire à base de polycaprolactone et de polychloroprène, son procédé de préparation et son utilisation notamment en orthopédie.**

(30) Priorité: **20.01.82 FR 8200855**

(43) Date de publication de la demande:
**24.08.83 Bulletin 83/34**

(45) Mention de la délivrance du brevet:
**02.10.85 Bulletin 85/40**

(84) Etats contractants désignés:
**BE DE FR GB IT NL**

(56) Documents cités:
**FR - A - 2 417 524**
**GB - A - 1 227 053**

(73) Titulaire: **LABORATOIRES D'HYGIENE ET DE DIETETIQUE L.H.D. Société Anonyme dite:, 38 avenue Hoche, F-75008 Paris (FR)**

(72) Inventeur: **Grouiller, Hervé, 1, rue d'Aval à Chevrey, F-21700 Arcenant (FR)**

(74) Mandataire: **Combe, André et al, CABINET BEAU DE LOMENIE 55 rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une nouvelle composition thermoplastique à mémoire à base de polycaprolactone et de polychloroprène. Elle concerne aussi le procédé de préparation de cette composition ainsi que son utilisation notamment en orthopédie en tant que moyen de contention.

On sait que la matière la plus utilisée pour immobiliser une partie du corps humain, et notamment un membre fracturé, est le plâtre. Il se trouve que, dans le domaine des structures orthopédiques, les bandages au plâtre constituent un moyen de contention présentant un grand nombre d'inconvénients. En effet, le plâtre est lourd et peu perméable à l'air, il absorbe et disperse les rayons X, ce qui gêne les diagnostics par radiographie, et il donne souvent lieu à des irritations de la peau.

On sait par ailleurs que, pour remplacer le plâtre, on a proposé dans le passé d'utiliser des structures orthopédiques en polycaprolactone. La polycaprolactone est une substance qui présente à la température ambiante une grande résistance à la flexion et un module d'élasticité élevé, mais qui a l'inconvénient à partir de 50° C de devenir molle et très collante. Si on chauffe la polycaprolactone en feuille à une température supérieure à environ 50° C, elle perd rapidement sa résistance mécanique et son élasticité, et elle ne peut même pas supporter son propre poids.

On vient de trouver de façon surprenante que, à partir de polycaprolactone (en abrégé PCL) et de polychloroprène (en abrégé PCP), on obtient une cmposition thermoplastique nouvelle constituant un produit industriel doué de mémoire élastique et qui présente des propriétés particulièrement intéressantes en ce qui concerne la résistance mécanique à la chaleur. Ce produit qui est notamment utile en tant que moyen orthopédique de contention, (i) est, par rappot au plâtre, d'un usage plus facile et d'un poids plus léger, et (ii) ne perd pas totalement, par rapport à la PCL, sa résistance mécanique, notamment à 50−60° C.

De plus, la solution technique que l'on propose ici pour résoudre le problème du remplacement du plâtre en orthopédie, est différente de celle que la Demanderesse préconise dans sa demande de brevet français No 82-00854 déposée le même jour que la présente invention et décrivant, en tant que moyen orthopédique de contention, une association de PCL et de polyuréthane dans laquelle le polyuréthane a été formé in situ.

La composition thermoplastique selon l'invention qui comprend de la PCL et du PCP et qui est notamment utile en tant que moyen orthopédique de contention est caractérisée en ce qu'elle renferme

A)   40 à 80 parties en poids de PCL, et
B)   20 à 100 parties en poids de polychloroprène.

Le cas échéant, cette composition thermoplastique peut renfermer un ou plusieurs adjuvants, notamment au moins un moyen choisi parmi l'ensemble constitué par les charges minérales, les colorants et les plastifiants. On pourra ainsi utiliser

C)   au plus 35 parties en poids de charge minérale, et/ou
D)   au plus 5 parties en poids d'agent plastifiant.

Les quantités préférées sont de 5 à 35 parties en poids pour le moyen C et de 1 à 2 parties en poids pour le moyen D, quand, bien entendu, lesdits moyens C et/ou D sont utilisés. Parmi les charges minérales qui conviennent, on peut notamment mentionner ZnO, $CaCO_3$, $TiO_2$, $Ta_2O_5$ et le talc; et parmi les plastifiants qui conviennent, on peut notamment mentionner l'acide stéarique qui joue également le rôle d'agent lubrifiant.

Le procédé de préparation que l'on préconise selon l'invention, qui comprend le mélange des moyens A et B, le cas échéant, en association avec un ou plusieurs adjuvants, puis le moulage en feuille de la composition résultante, est caractérisé en ce qu'il comprend

1°)   le mélange des moyens A et B à une température comprise entre 40 et 120° C, pendant 1 à 10 min, sous agitation, à une vitesse angulaire comprise entre 50 et 300 tours/minute, puis

2°)   le moulage sous pression de la composition résultante à une température supérieure ou égale à 60° C (et de préférence comprise entre 60 et 100° C) pendant 10 à 30 min, pour obtenir une feuille ayant une épaisseur comprise entre 1 et 7 mm (et de préférence entre 1 et 4,5 mm).

De façon avantageuse, on met en oeuvre le stade 1° à une température comprise entre 60 et 120° C après avoir introduit le PCP dans la PCL fondue.

Le PCP que l'on utilise est avantageusement un polymère du type trans-trans, c'est-à-dire un trans-polychloroprène, par exemple un produit commercialisé par la Sté DUPONT DE NEMOURS sous la nomenclature de »NEOPRENE AD«.

La PCL que l'on utilise est avantageusement un polymère ayant un poids moléculaire de l'ordre de 35 000 environ à 40 000 environ.

Selon un mode particulier de mise en oeuvre de l'invention, il est possible d'associer aux moyens A et B un polyuréthane préparé in situ comme indiqué dans la demande précitée par réaction dans des conditions sensiblement stoechiométriques d'un polyol, notamment du type polyéther-polyol ou polyester-polyol, avec un polyisocyanate, notamment un di-, tri- ou tétra-isocyanate tel que par exemple le 2,4-toluènediisocyanate, le 2,6-toluènediisocyanate et le toluènediisocyanate commercial renfermant 80% en poids d'isomère 2,4 et 20% en poids d'isomère 2,6. Selon ce mode particulier, on utilise au plus 40 parties en poids de polyuréthane en association avec les moyens A et B, et, le cas échéant, les moyens C et/ou D.

D'autres avantages et caractéristiques de l'invention seront mieux compris à la lecture qui va suivre d'exemples de préparation nullement limitatifs, mais donnés à titre d'illustration.

Exemples 1 à 4

Dans un malaxeur étanche à pales chauffé à 60°C, on introduit d'abord la PCL, on ajoute ensuite une faible quantité d'agent plastifiant (acide stéarique). Lorsque le mélange est fondu et homogène, on ajoute la quantité requise de PCP (commercialisé sous la dénomination de »NEOPRENE AD 30« par la Société DUPONT DE NEMOURS) et, le cas échéant, du TiO₂ (ayant une granulométrie inférieure ou égale à 100 μ). Le mélange résultant est maintenu sous agitation à 150 tours/minute, à une température de 60°C pendant 5 minutes. Le mélange chaud est retiré du malaxeur et est coulé dans des moules carrés (de 0,5 m de côté) et pressé à 100°C pendant 20 minutes. On refroidit jusqu'à 15—20°C au moyen d'un circuit d'eau froide. Les feuilles moulées ainsi obtenues, qui ont une épaisseur de 2,5 à 4 mm, ont les compositions suivantes:

Exemple 1:
70 parties en poids de PCL, 30 parties en poids de PCP, 20 parties en poids de TiO₂ et 2 parties en poids d'acide stéarique;

Exemple 2:
60 parties en poids de PCL, 40 parties en poids de PCP et 2 parties en poids d'acide stéarique;

Exemple 3:
50 parties en poids de PCL, 50 parties en poids de PCP, 20 parties en poids de TiO₂ et 2 parties en poids d'acide stéarique;

Exemple 4:
70 parties en poids de PCL, 30 parties en poids de PCP et 2 parties en poids d'acide stéarique.

Les feuilles selon l'invention, et en particulier celles des exemples 1 à 4, se prêtent aisément au moulage sur la portion de la surface du corps qui a besoin d'un moyen orthopédique de contention, sans qu'il soit nécessaire de forcer pour les étirer. On les réchauffe par exemple à 60°C, notamment par immersion dans de l'eau à cette température avant de les appliquer sur la peau. A chaud, elles sont autocollantes et adhèrent bien les unes aux autres sur simple pression. Après refroidissement, elles présentent une bonne résistance au décollement, une grande rigidité, la découpe se faisant alors aux ciseaux.

La composition selon l'invention peut être également utile dans d'autres applications, par exemple dans le domaine des matériaux de scellement d'insert.

## Revendications

1. Nouvelle composition thermoplastique comprenant de la polycaprolactone et du polychloroprène et notamment utile en tant que moyen orthopédique de contention, caractérisée en ce qu'elle renferme

A) 40 à 80 parties en poids de polycaprolactone, et
B) 20 à 100 parties en poids de polychloroprène.

2. Composition thermoplastique selon la revendication 1, caractérisée en ce qu'elle renferme en outre un moyen choisi parmi l'ensemble constitué par les charges minérales, les colorants, les plastifiants et leurs mélanges.

3. Composition selon la revendication 2, caractérisée en ce qu'elle renferme au plus 35 parties en poids de charge minérale.

4. Composition selon la revendication 2, caractérisée en ce qu'elle renferme au plus 5 parties en poids d'agent plastifiant.

5. Composition selon la revendication 1, caractérisée en ce qu'elle renferme en outre du polyuréthane formé in situ en quantité inférieure ou égale à 40 parties en poids.

6. Procédé de préparation d'une composition selon l'une quelconque des revendications 1 à 5, présentée sous forme de feuille, ledit procédé étant caractérisé en ce qu'il comprend

1°) le mélange de 40 à 80 parties en poids de polycaprolactone avec 20 à 100 parties en poids de polychloroprène à une température comprise entre 40 et 120°C, pendant 1 à 10 min, sous agitation, à une vitesse angulaire comprise entre 50 et 300 tours/minute, puis

2°) le moulage sous pression de la composition résultante à une température supérieure ou égale à 60°C (et de préférence comprise entre 60 et 100°C) pendant 10 à 30 min, pour obtenir une feuille ayant une épaisseur comprise entre 1 et 7 mm (et de préférence entre 1 et 4,5 mm).

## Patentansprüche

1. Neue thermoplastische Zusammensetzung mit Polycaprolacton und Polychloropren, insbesondere als orthopädisches Stützmittel, dadurch gekennzeichnet, daß sie umfaßt:

A) 40 bis 80 Gew.-Teile Polycaprolacton und
B) 20 bis 100 Gew.-Teile Polychloropren.

2. Thermoplastische Zusammensetzung nach Anspruch 1, dadurch gekennzeichnet, daß sie außerdem ein Mittel umfaßt, das ausgewählt ist unter der Gesamtheit, die gebildet ist durch mineralische Füllstoffe, Farbstoffe, Plastifikatoren und ihre Gemische.

3. Zusammensetzung nach Anspruch 2, dadurch gekennzeichnet, daß sie höchstens 35 Gew.-Teile an mineralischem Füllstoff umfaßt.

4. Zusammensetzung nach Anspruch 2, dadurch gekennzeichnet, daß sie höchstens 5 Gew.-Teile an Plastifikator umfaßt.

5. Zusammensetzung nach Anspruch 1, dadurch gekennzeichnet, daß sie außerdem Polyurethan umfaßt, das in situ in einer Menge von kleiner oder gleich 40 Gew.-Teilen hergestellt ist.

6. Verfahren zum Bereiten einer Zusammensetzung nach einem der Ansprüche 1 bis 5 als Folie, wobei das Verfahren dadurch gekennzeichnet ist, daß es aufweist:

1) das Mischen von 40 bis 80 Gew.-Teilen Polycaprolacton mit 40 bis 100 Gew.-Teilen Polychloropren bei einer Temperatur zwischen 40 und 120°C während 1 bis 10 min unter Umrühren mit einer Winkelgeschwindigkeit zwischen 50 und 30 U/min, dann

2) das Druckgießen der resultierenden Zusammensetzung bei einer Temperatur von über oder gleich 60°C (vorzugsweise zwischen 60 und 100°C) während 10 bis 30 min zur Erzielung einer Folie mit einer Dicke zwischen 1 und 7 mm (vorzugsweise zwischen 1 und 4,5 mm).

**Claims**

1. Novel themoplastic composition comprising polycaprolactone and polychloroprene, and particularly useful as orthopedic containing means, characterized in that it comprises

A) 40 to 80 parts by weight of polycaprolactone, and
B) 20 to 100 parts by weight of polychloroprene.

2. Thermoplastic composition according to claim 1, characterized in that it further comprises a means selected from the group constituted by the mineral fillers, the dying agents, the plastifying agents and mixtures thereof.

3. Composition according to claim 3, characterized in that it contains at the most 35 parts by weight of mineral filler.

4. Composition according to claim 2, characterized in that it contains at the most 5 parts by weight of plastifying agent.

5. Composition according to claim 1, characterized in that it further contains polyurethane formed in situ in a quantity lower than or equal to 40 parts by weight.

6. Preparation process of a composition according to any one of claims 1 to 5, presented in sheet form, said process being characterized in that it comprises

1) the mixture of 40 to 80 parts by weight of polycaprolactone with 20 to 100 parts by weight of polychloroprene at a temperature varying between 40 and 120°C, for 1 to 10 minutes, under stirring, at an angular speed between 50 and 300 revs/minute, then

2) the molding under pressure of the resulting composition at a temperature higher than or equal to 60°C (and preferably between 60 and 100°C) for 10 to 30 minutes to obtain a sheet of thickness varying between 1 and 7 mm (and preferably between 1 and 4.5 mm).